# EUROPEAN PATENT SPECIFICATION

(11) **EP 2 090 184 B1**
(45) Date of publication and mention of the grant of the patent: **03.08.2011**
(21) Application number: 09152728.3
(22) Date of filing: 12.02.2009
(51) Int. Cl.: A41D 13/08, A61F 5/01, G06F 3/039

(54) **Wrist pad**
Handgelenksauflage
Repose-poignet

(30) Priority: 14.02.2008 US 65983 P
(43) Date of publication of application: 19.08.2009
(73) Proprietor: Diaz, Adalberto R., Cinnaminson, New Jersey 08077 (US); Fernandez, Juan, Towaco, New Jersey 070782 (US)
(72) Inventor: Diaz, Adalberto R., Cinnaminson, New Jersey 08077 (US); Fernandez, Juan, Towaco, New Jersey 070782 (US)
(74) Representative: Carpintero Lopez, Francisco

(56) References cited:
- WO-A2-2006/077572
- US-A1- 2002 062 095
- US-A1- 2004 133 137
- US-B1- 6 517 501

## Description

### Field of the Invention

The invention relates to wrist pads, in particular for use by data entry personnel and others who spend long periods using keyboards and computer mice. The goal of wrist pads and related devices is the prevention of repetitive motion injuries.

### Background of the Invention

The invention β defined in claim 1 and relates to a wrist pad having a pad extension US-B1-6517501 discloses one kind of wrist pad or similar, with a pad extension. The pad extension of the present invention prevents the user's wrist from flexing downward during use, reducing wrist strain and improving comfort to the user. The prior art above does not teach multiple layers of cushioning materials and a massaging vibrator, which extend into the palm area of the hand. None of the prior art addresses a unique feature of the present invention, which is the presence of motor-driven vibrators in the wrist pad to massage the wrist and lower palm.

The present invention contains features that, when combined, provide more relief to the hand of an operator than any of the prior art devices described above. For instance, the foam pad conforms slightly and provides some cushioning to the wrist, while the gel pad provides further protection by adding more cushioning and yield. The vibrations emanating through the pads massage tired muscles, but the wrist is still supported in a way that is natural to the shape and movement of the hand.

Although this invention is described primarily for users operating a computer keyboard or mouse, it can also be used in other applications, such as but not limited to, use of a manual typewriter, while driving a car with hands resting on the steering wheel, when knitting or crocheting, or for any type of work or hobby where the hand or wrists

are strained, held in the same position for periods of time, or are engaged in repetitive motions, or just to massage a hand or wrist after activity of any kind or for any reason at all.

It is an object of the invention to provide an improved wrist pad that increases comfort for the user and reduced wrist strain.

It is another object of the invention to teach a wrist pad having a pad extension that provides additional support to a user's wrist.

It is a further object of the invention to provide a vibrating mechanism which relaxes and sooths the users wrist.

These and other objects will become clear from the description of the invention below.

### Summary of the Invention

An article of manufacture comprising a wrist pad having a pad base and a pad extension and a wrist strap. The pad extension extends towards the palm from the wrist pad base. The article may also contain a vibrating mechanism and a support. The preferred vibrating mechanism is one or more coin motors. The support is preferably constructed of plastic.

### Brief Description of the Drawings

Fig. 1 is a perspective view from the right of the wrist pad used on the left hand with a computer mouse.
Fig. 2 is a perspective view from the right of the wrist pad used on the left hand.
Fig. 3 is a front perspective view of the wrist pad with wrist straps unattached.
Fig. 4 is a top perspective view of the wrist pad with wrist straps unattached.
Fig. 5 is a front perspective view looking slightly down at wrist pad with wrist straps attached.
Fig. 6 is a perspective view 45 degrees to the side looking slightly down at wrist pad with wrist straps unattached.
Fig. 7 is an exploded top view of wrist pad.
Fig. 8 shows a top view of an alternate embodiment of the invention.
Fig. 9 shows a top perspective view of an alternate embodiment of the invention.

### Description of the Preferred Embodiments

The preferred embodiment of the present invention will now be described with reference to the drawings. Identical elements in the various figures are identified with the same reference numerals.

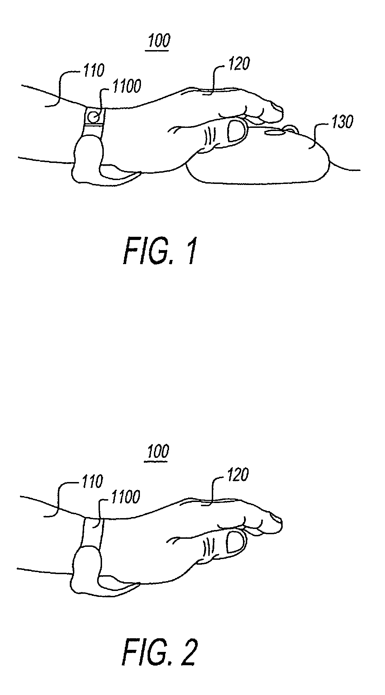

Fig. 1 shows the wrist pad 100 on the wrist 110 of the user. The hand of the user 120 is shown holding the mouse 130, in a way that is typical when conducting data entry on a computer. Gel pad 100 is attached to the user's wrist 110 by the right strap 1100.
Fig. 2 illustrated the user's hand 120 with gel pad 100 on wrist 110. Strap 1100 holds the gel pad 100 onto the user's wrist 110. Figure 2 shows the user wearing the wrist pad on the user's left hand, but the invention may be worn on the right hand as well.

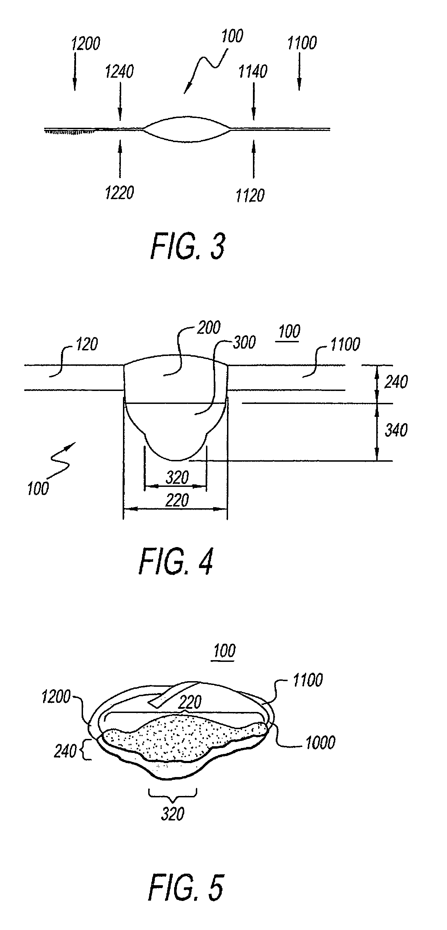

Fig. 3 shows the wrist pad 100 unattached to a wrist. The wrist pad 100 is shown from a front view, with right strap 1100, which has a bottom 1120 and a top 1140. Fig. 3 also shows left strap 1200, which has a bottom 1220 and a top 1240.
Fig. 4 shows a top view of the wrist pad 100 unattached to a wrist. The wrist pad has a pad base 200 which rests under the wrist, and a wrist pad extension 300 which extends into the palm of the hand. The pad extension of the present invention prevents the users wrist from flexing downward during use, reducing wrist strain and improving comfort to the user. Wrist pad extension 300 has width 320 and a length 340. Wrist pad extension 300 can be any shape but is preferably conformed to fit into the palm of the users hand, and will preferably have a generally conical shape as seen in Fig 4. Wrist pad extension has length 340, which is at leastl.3 cm and preferably from 1.9 to 5.1 cm, and most preferably 2.5 cm to 5.1 cm in length. Wrist pad extension 300 also has a width 320, which is from 1.3 to 7.5 cm, and may vary in width to a taper or conical shape as seen in figure 4. Wrist pad base 200 has a width 220 and a length 240. Wrist pad base width 220 can be from 6.4 to 11.4 cm and is preferably about 8.9 cm , and wrist pad base length 240 is from 1.9 to 5.1 cm with 3.8 cm being preferred. The total length of the of the invention 225, as seen in Fig. 4, will vary from 20.3 to 30.5 cm, which includes the strap lengths, with a preferred length of about 25.4 cm. The dimensions are above are mostly illustrative and could be varied depending on the size of the user's hand. Commercial models of the invention could, for example, be sold in small, medium and large sizes with the basic dimensions altered accordingly. Fig. 4 also shows right strap 1100 and left strap 1200.

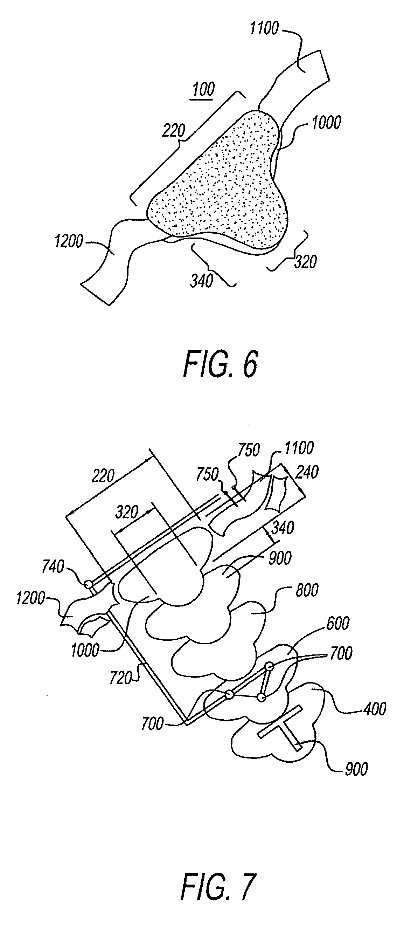

Fig. 5 and Fig. 6 illustrate the wrist pad 100, Fig. 5 with straps attached to each other, Fig. 6 with the straps not attached. The wrist pad 100 is not on the user's wrist. Fig. 5 and Fig. 6 show right strap 1100 and left strap 1200, the canvas cover of the wrist pad 1000, the pad base width 220, the pad base length 240, the pad extension width 320, and the pad extension length 340. In a preferred embodiment, the pad base width 220 is greater than the pad extension width 320, with the pad extension width 320 being about ½ the pad base width 220.
Fig. 7 shows a preferred embodiment of the invention in which the wrist pad is constructed in layers of material which have the same shape. Each layer has a pad base 200 and a pad extension 300. The pad base 200 has pad base width 220 and pad base length 240. The pad extension 300 has a pad extension width 320 and a pad extension length 340. In Fig. 7, and in a preferred embodiment of the invention, the pad extension width 320 is approximately half of pad base width 220. The pad extension length 340 is at least half an inch. Typically, the pad base 200 and the pad extension 300 are made in a single piece, but could also be made from separate pieces and attached together using conventional means, such as glue or sewing.

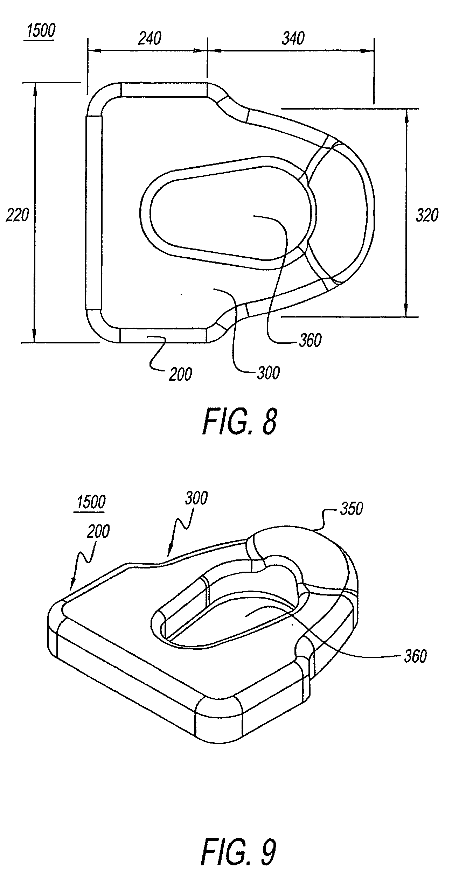

Figs 8 and 9 show views of an alternate embodiment of the wrist pad 1500 unattached to a wrist. The wrist pad has a pad base 200 which rests under the wrist, and a wrist pad extension 300 which extends into the palm of the hand. The pad extension prevents the users wrist from flexing downward during use, reducing wrist strain and improving comfort to the user. Wrist pad extension 300 has width 320 and a length 340 and wrist pad base 200 has a width 220 and a length 240.

Wrist pad extension 300 can be any shape but is preferably conformed to fit into the palm of the users hand, and will preferably have a generally conical shape. Note also in Fig. 8 that wrist pad extension 300 may have a raised lip 350 that can give further support to the users hand. Raised lip can be beveled, as shown in the drawing or it can be any suitable shape, such as rectangular or triangular. Wrist pad extension has length 340, which is at least 1.3 cm and preferably from 1.9 to 5.1 cm, and most preferably 2.5 cm to 5.1 cm in length. Wrist pad extension 300 also has a width 320, which is from 1.3 to 7.5 cm, and may vary in width to a taper or conical shape as seen in figure 4. Wrist pad base 200 has a width 220 and a length 240. Wrist pad base width 220 can be from 6.4 to 11.4 cm and is preferably about 8.9 cm, and wrist pad base length 240 is from 1.9 to 5.1 cm with 3.8 cm being preferred.

Alternate embodiment 1500 could be inserted in bottom and top canvas covers which are in turn attached to straps. Alternatively, straps could be attached directly to the invention 1500. Embodiment 1500 is preferably molded from gel, plastic, or rubber, but could be any material. The embodiment 1500 could be a solid piece or could be fabricated from a honey combed material.

The total length of the of the invention 225, as seen in Fig. 4, will vary from 20.3 to 30.5 cm, which includes the strap lengths, with a preferred length of about 25.4 cm. The dimensions are above are mostly illustrative and could be varied depending on the size of the user's hand

In this embodiment shown in Figs 8 and 9, the pad base 200 and wrist pad extension 300 have material removed to form a cavity 360. The cavity 360 makes the wrist pad lighter. Also, if wrist straps are attached directly to the pad base 200, cavity 360 allows air to circulate to the user's palm, making the pad more comfortable.

The preferred embodiment is constructed from five layers. A larger or smaller number of layers or no layers could be used to construct the invention. The layers may all be the same shape, may all be different shapes, or some could be the same shape while others are different shapes. A wide variety of materials may be used for the various layers, including but not limited to gels, made from materials including but not limited to, natural materials such as aloe vera, man made materials such as petroleum gels, or any combination thereof. Other materials for the wrist pad include but are not limited to foams, beads, soil, seeds, elastomers, plastics, rubbers, feathers, canvas, leather, fabrics, wood, paper, cardboard, glass, fiberglass, sand, metals, animal or plant components, fluids, such as but not limited to, oils or water or combinations thereof. The purpose of the multiple layers is to separate the inner components from one another to reduce wear and tear. The advantages of multiple layering is also to enhance users comfort. In addition, multiple padding serves to protect the users hand and or wrist in the event that the unit is exposed to extreme heat or cold.

The wrist pad 100 may be formed as a single piece in the preferred embodiment. Alternatively, it is possible that the wrist pad 100 might be formed as multiple pieces.

As illustrated in Fig. 7, the preferred embodiment may consist of a bottom canvas cover 400, plastic support 500, cotton pad 600, vibrating mechanism 700, gel pad 800, foam pad 900, top canvas cover 1000 right wrist strap 1100 and left wrist strap 1200. The plastic support 500 may be fastened to the bottom canvas cover 400 in any way, including but not limited to gluing, or sewing. Additionally, the plastic support 500 could be constructed with small holes so it could be more readily fastened to bottom canvas cover 400. The plastic support could be replaced by a support made of other materials, including but not limited to, metal or wood. Plastic support 500 supports wrist pad extension 300, and may be shaped in any manner that gives additional support to the user's wrist. Any of the components shown in Fig. 7 can be made from any of the materials described above.

Vibrating mechanism 700 may be one or more vibrating coin motors fastened into the cotton pad 600 by methods such as, but not limited to, sewing or gluing. Also other kinds of vibrating mechanisms could be used in place of vibrating coin motors, such as but not limited to miniature vibrating cylinder motors. Wires 720 may be connected to the vibrating coin motors 700 and lead to the on/off switch 740 and then onto the batteries 740, which may be affixed to the right wrist strap 1100. Alternatively the vibrating mechanism power source and switch could be completely contained in the wrist pad 100. Other alternative arrangements for powering the vibrating coin motors 700 are possible, such as but not limited to rechargeable and solar powered units. Preferably, the unit is rechargeable and economical for the user. The rechargeable unit would have a LED light to indicate when the unit is fully charged and an automatic charge shut off. This will avoid overcharging and increase the batteries life.

The bottom canvas cover 400, cotton pad 600, gel pad 800, foam pad 900, and top cover 1000 may be fastened together along their matching perimeters or at any place in their bodies by methods such as, but not limited to, gluing or sewing. The layers may be joined together in such a way as to allow for wires to emerge from the wrist pad.

The right wrist strap 1100 and the left wrist strap 1200 may be made of rectangular pieces of elastic. Other materials that could be used for the right wrist strap 1100 and the left wrist strap 1200 include but are not limited to, fabrics such as leather, cotton, or nylon., or other materials such as but not limited to paper, plastic, metals, fiberglass, or wood. The right wrist strap 1100 has a right bottom surface 1120 and a right top surface 1140. The left wrist strap 1200 has a left bottom surface 1220 and a left top surface 1240 Hook and loop fasteners may be attached to the right top surface 1140 and a left bottom surface 1220 forming closing and adjustment mechanism for the wrist straps. Other kinds closing and adjustment mechanisms include but are not limited to belt buckles, snaps, buttons, and slip buckles. Devices for containing batteries may be attached to the right wrist strap 1200 to power a vibrating mechanism. The right wrist strap 1100 and the left wrist strap 1200 may be fastened to wrist pad 100 by methods including but not limited to gluing or sewing.

A wide variety of shapes can be accommodated by this specification. Compare Fig. 4 to Fig 7. In Fig.4 the pad base 200 is almost rectangular as compared to the more rounded shape of the pad base 200 in Fig. 7. In Fig. 4 the pad extension 300 had a complex curvature as compared to the simple curvature of the pad extension 300 in Fig. 7.

In another embodiment, the strap is basically a metal band which is curved a certain way so when it is bent it will coil and wrap itself around the user's wrist. In this version the slap bracelet has an outer silicone rubber coating to provide comfort. This silicone rubber coating also would allow for the co-molding of the wrist pad and bracelet into one piece as opposed to the mockup sample in the pictures that is two pieces joined together.

The gel wrist pad is attached to the slap bracelet through a backing which acts like a belt buckle and the slap bracelet is inserted into this opening and it allows the wrist pad to slide side to side on the bracelet so the user can position it where they want it.

Something unique about the backing is that it is made of a special material which allows it to easily glide on just about any surface.

Although this invention has been described with a certain degree of particularity, it is to be understood that the present disclosure has been made only by way of illustration and that numerous changes in the details of construction and arrangement of parts may be resorted to without departing from the scope of the invention.

## Claims

1. An article of manufacture, comprising:
a wrist pad (100, 1500) having a pad base (200) and a pad extension (300); and
a wrist strap (1100, 1200);
**characterised in that**
the pad base has at least one vibrating mechanism (700).

2. The article of claim 1, wherein the pad base (200) has a width (220), and the pad extension (300) has a width (320), and the pad base width is greater than the pad extension width.

3. The article of claim 1 to 2, wherein, the pad base (200) has a width (220), and the pad extension (300) has a width (320), and the pad extension width is approximately half the width of the pad base.

4. The article of claims 1 to 3, wherein the pad extension (300) has a length (340), and the length is at least 1.3 cm.

5. The article of claims 1 to 4, wherein the pad base (200) and the pad extension (300) are formed as a single piece.

6. The article of claims 1 to 5, wherein the pad base (200) and pad extension (300) have a gel pad (800).

7. The article of claims 1 to 6, wherein the wrist strap (1100, 1200) is adjustable with hook and loop fasteners.

8. The article of claims 1 to 7, wherein the pad base (200) and pad extension (300) have a plastic support (500).

9. The article of claims 1 to 8, wherein the pad base (200) and pad extension (300) have material removed to form a cavity (360).

10. The article of claims 1 to 9, wherein the pad extension has a raised lip (350).

## Patentansprüche

1. Ein Produkt,enthaltend:
eine Handgelenksauflage (100, 1500) mit einer Auflagebasis (200) und einer Auflageerweiterung (300); und
einem Handgelenksband (1100, 1200);
**dadurch gekennzeichnet, dass**
die Auflagebasis mindestens einen Vibrationsmechanismus aufweist (700).

2. Das Produkt aus Anspruch 1, wobei die Auflagebasis (200) eine Breite (220) aufweist, und die Auflageerweiterung (300) eine Breite (320) aufweist, und die Breite der Auflagebasis größer ist als die Breite der Auflageerweiterung.

3. Das Produkt aus Anspruch 1 bis 2, wobei die Auflagebasis (200) eine Breite (220) aufweist, und die Auflageerweiterung (300) eine Breite (320) aufweist, und die Breite der Auflageerweiterung ungefähr die Hälfte der Breite der Auflagebasis beträgt.

4. Das Produkt aus Anspruch 1 bis 3, wobei die Auflageerweiterung (300) eine Länge (340) aufweist, und die Länge mindestens 1.3 cm beträgt.

5. Das Produkt aus Anspruch 1 bis 4, wobei die Auflagebasis (200) und die Auflageerweiterung (300) einstückig ausgebildet sind.

6. Das Produkt aus Anspruch 1 bis 5, wobei die Auflagebasis (200) und die Auflageerweiterung (300) ein Gelpolster (800) aufweisen.

7. Das Produkt aus Anspruch 1 bis 7, wobei das Handgelenksband (1100, 1200) mit Klettverschlüssen einstellbar ist.

8. Das Produkt aus Anspruch 1 bis 7, wobei die Auflagebasis (200) und die Auflageerweiterung (300) eine Kunststoffstütze (500) aufweisen.

9. Das Produkt aus Anspruch 1 bis 8, wobei aus der Auflagebasis (200) und der Auflageerweiterung (300) Material entfernt wurde um eine Aushöhlung (360) zu bilden.

10. Das Produkt aus Anspruch 1 bis 9, wobei die Auflageerweiterung eine angehobene Nase (350) aufweist.

## Revendications

1. Article manufacturé comprenant :
un repose-poignet (100, 1500) ayant une base de coussinet (200) et une extension de coussinet (300) ; et
une sangle de poignet (1100, 1200)
**caractérisé en ce que** :
la base de coussinet a au moins un mécanisme vibrant (700).

2. Article selon la revendication 1, dans lequel la base de coussinet (200) a une largeur (220), et l'extension de coussinet (300) a une largeur (320), et la largeur de la base de coussinet est supérieure à la largeur de l'extension de coussinet.

3. Article selon la revendication 1 ou 2, dans lequel, la base de coussinet (200) a une largeur (220) et l'extension de coussinet (300) a une largeur (320), et la largeur de l'extension de coussinet représente approximativement la moitié de la largeur de la base de coussinet.

4. Article selon les revendications 1 à 3, dans lequel l'extension de coussinet (300) a une longueur (340) et la longueur est d'au moins 1,3 cm.

5. Article selon les revendications 1 à 4, dans lequel la base de coussinet (200) et l'extension de coussinet (300) sont formées d'un seul tenant.

6. Article selon les revendications 1 à 5, dans lequel la base de coussinet (200) et l'extension de coussinet (300) ont un coussinet en gel (800).

7. Article selon les revendications 1 à 6, dans lequel la sangle de poignet (1100, 1200) est ajustable avec des fixations à Velcro.

8. Article selon les revendications 1 à 7, dans lequel la base de coussinet (200) et l'extension de coussinet (300) ont un support en plastique (500).

9. Article selon les revendications 1 à 8, dans lequel du matériau a été retiré de la base de coussinet (200) et de l'extension de coussinet (300) afin de former une cavité (360).

10. Article selon les revendications 1 à 9, dans lequel l'extension de coussinet a une lèvre relevée (350).
